# EUROPÄISCHE PATENTSCHRIFT

(11) **EP 0 588 222 B1**
(45) Veröffentlichungstag und Bekanntmachung des Hinweises auf die Patenterteilung: **26.02.1997**
(21) Anmeldenummer: 93114381.2
(22) Anmeldetag: 08.09.1993
(51) Int. Cl.: C07D 319/12

(54) **Verfahren zur Herstellung von Glykolid und Lactid**
Process for the preparation of glycolid and lactid
Procédé de préparation de glycolide et lactide

(30) Priorität: 16.09.1992 DE 4230951; 03.06.1993 DE 4318456
(43) Veröffentlichungstag der Anmeldung: 23.03.1994
(73) Patentinhaber: BASF Aktiengesellschaft, 67063 Ludwigshafen (DE)
(72) Erfinder: Hoessel, Peter, Dr., D-67105 Schifferstadt (DE); Hoffmann, Gerhard, Dr., D-67166 Otterstadt (DE); Braun, Frank, D-67150 Niederkirchen (DE); Kratz, Detlef, Dr., D-69120 Heidelberg (DE); Brudermueller, Martin, Dr., D-68165 Mannheim (DE); Witzel, Tom, Dr., D-67069 Ludwigshafen (DE)

(56) Entgegenhaltungen:
- EP-A- 0 261 572
- DE-B- 1 234 703
- US-A- 4 650 851

## Beschreibung

Die vorliegende Erfindung betrifft ein neues Verfahren zur Herstellung von Glycolid (Ia; 1,4-Dioxan- 2,5-dion) oder von Lactiden (Ib; 3,6-Dimethyl-1,4-dioxan-2,5-dione) 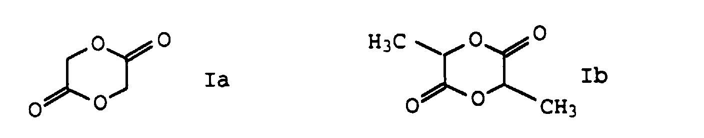 durch thermisch-katalytische Spaltung von Kondensationsprodukten der Glycolsäure (IIa) bzw. einer α-Hydroxypropionsäure (IIb) unter destillativer Entfernung 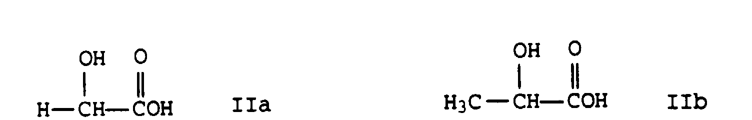 von Ia bzw. Ib aus dem Reaktionsgemisch, Überführung des Destillates durch Abkühlen in die feste Phase und Reinigung des so erhaltenen Rohproduktes.

Glykolid und Lactide sind die Ausgangsstoffe für hochmolekulare Polyglycolide bzw. Polylactide. Diese Polymeren finden u.a. Verwendung als resorbierbare chirurgische Nahtmaterialien.

Die an sich bekannte technische Herstellung von Glycolid bzw. Lactiden für die Weiterverarbeitung zu Polymeren geht im allgemeinen von den monomereno α-Hydroxycarbonsäuren Glycolsäure und Milchsäure aus, wobei diese in der D-oder L-Form sowie als racemisches Gemisch vorliegen kann. Diese Säuren werden durch Erhitzen zu oligomeren Estern kondensiert, welche dann thermisch in Gegenwart von Katalysatoren in die Dimeren Ia bzw. Ib gespalten und abdestilliert werden.

Die Reinigung des so erhaltenen Rohproduktes von Verunreinigungen (überwiegend Oligomere der eingesetzten Ausgangssäuren und diese Säuren selbst), erfolgte bisher durch Umkristallisation aus verschiedenen Lösungsmitteln, z.B. iso-Propanol (EP-A 261 572), tert.-Amylalkohol (DE-A 1808939), Tetrachlorkohlenstoff (DE-A 1234703) oder Ethylacetat (US-A 4727163).

Diese Reinigungsverfahren sind jedoch unbefriedigend, da die Produkte nicht immer auskristallisieren, sondern mitunter als Öl anfallen, und weil die Umkristallisation in der Regel mehrfach durchgeführt werden muß, um Produkte in solch hoher Reinheit zu erhalten, die für die Herstellung hochmolekularer Polymere unerläßlich ist.

Außerdem können Lösungsmittel wie Alkohole unter Spaltung von Ia bzw. Ib zu Estern der Glykolsäure IIa bzw. α-Hydroxypropionsäure IIb führen.

Die WO 92/00974 betrifft die Reinigung von Rohlactid in einem Zweiphasensystem aus Wasser und einem Keton, Ether oder Ester. Bei dieser Arbeitsweise kann es durch Reaktion des Wassers mit Lactid zu Verlusten am Wertprodukt kommen.

Es bestand daher die Aufgabe, ein Verfahren zur Verfügung zu stellen, das es erlaubt, die Rohprodukte, die nach dem oben beschriebenen Verfahren hergestellt werden, auf einfache Weise zu reinigen.

Demgemäß wurde das oben genannte Verfahren gefunden, das dadurch gekennzeichnet ist, daß man das Rohprodukt mit einem Ether mit 4 bis 12 Kohlenstoffatomen wäscht.

Bei den erfindungsgemaß zu verwendenden Ethern handelt es sich um Ether mit insgesamt 4 bis 12 Kohlenstoffatomen. Es kommen aliphatische Ether wie Diethylether, Di-iso-propylether, Di-n-butylether und Methyl-tert.-butylether in Betracht, weiterhin cycloaliphatische Ether wie Tetrahydrofuran, araliphatische Ether wie Anisol, aliphatische Polyether wie Glykoldimethylether und Glykoldi-n-butylether und cyclische Polyether wie Dioxan. Bevorzugt sind aliphatische Ether mit 4 bis 7 Kohlenstoffatomen, insbesondere Diethylether, Di-iso-propylether und Methyl-tert.-butylether.

Die Herstellung des Glycolids und der Lactide kann durch Erhitzen von wäßrigen Lösungen von IIa bzw. IIb erfolgen. Dabei wird in einer bevorzugten Ausführungsform Wasser abdestilliert. Die Sauren IIa bzw. IIb oligomerisieren und werden dann in die Dimeren der Säuren, nämlich Ia bzw. Ib gespalten. Für diese Spaltungsreaktion werden Katalysatoren verwendet, z.B. Zinn, Zinnhalogenide oder Zinnsalze von Carbonsäuren wie Sn(II)-2-Ethylhexanoat (EP-A 261 572), Titanalkylate (DE-A 1234703) und Antimonoxid oder Antimonhalogenide (Prep. Meth. of Polymer Chem., 2. Auflage, 1968, S. 363). Glycolid bzw. die Lactide werden dann abdestilliert, gegebenenfalls bei gleichzeitigem Durchleiten eines inerten Gases wie Stickstoff (z.B. US-A 4 835 293), und zum festen Rohprodukt abgekühlt.

Das durch die beschriebene Reaktion erhaltene rohe Glycolid bzw. Lactid kann direkt auf einem Filter mit einem Ether gewaschen werden oder auch zuvor in dem Ether aufgeschlämmt und dann filtriert werden.

Im allgemeinen verwendet man zur Reinigung von 1 kg des Rohproduktes 0,1 bis 100, vorzugsweise 0,3 bis 3 kg Ether.

Es kann bevorzugt im Bereich von -78 bis 50°C, besonders bevorzugt bei 0 bis 30°C gearbeitet werden. Es ist aber am einfachsten, die Reinigung bei Raumtemperatur vorzunehmen.

Der Ether löst die Verunreinigungen, und es verbleibt nach Abtrennung der Etherphase ein farbloses Produkt. Die Etherphase kann zur Trockne eingeengt werden, und der dabei anfallende Feststoff kann wieder zur Herstellung des Glycolids bzw. Lactids eingesetzt werden.

Das erfindungsgemäße Verfahren erlaubt in technisch einfacher Weise, Glycolid und Lactide in hoher Reinheit herzustellen.

Besondere Bedeutung hat das Verfahren für optisch aktives [L,L]-Lactid und [D,D]-Lactid, das aus optisch aktiver D- bzw. L-Milchsäure hergestellt wird. Das erfindungsgemäße Verfahren ermöglicht es, das Verhältnis der besonders gesuchten optisch aktiven Lactide gegenüber der meso-Form durch den Reinigungsschritt zu erhöhen.

### Beispiele

### Beispiel 1

### Herstellung von Rohlactid

88 gew.-%ige wäßrige L-Milchsäure wurde bei 130°C über eine Destillationsbrücke entwässert, bis kein Wasser mehr kondensierte. Dann wurde die Sumpftemperatur in 8 h auf 200°C erhöht, um das Reaktionswasser zu entfernen. Die so erhaltene Oligomilchsäure wurde mit 1,5 Gew.-% Sn(II)-Ethylhexanoat bei 250°C gespalten. Das Produkt (Rohlactid) fiel als Kristallbrei an.

### Allgemeine Verfahrensvorschrift

90 g Rohlactid (s. Beispiel 1) wurden in 30 g Ether gerührt und auf 0°C abgekühlt. Der farblose Feststoff wurde abfiltriert und mehrmals mit insgesamt 30 g des entsprechenden Ethers gewaschen. Die Versuchsergebnisse können der Tabelle entnommen werden.

| Ether | Gehalt des Feststoffs an Lactid [%] | Verhältnis opt. aktives Lactid : meso-Form | |
|---|---|---|---|
| | | im Feststoff | in Mutterlauge |
| Tetrahydrofuran | 98,5 | 19:1 | 2,1:1 |
| Dioxan | 94,2 | 32:1 | 3,2:1 |
| Glykoldimethylether | 96,7 | 13:1 | 2,3:1 |
| Glykoldi-n-butylether | 100 | 3:1 | 0,5:1 |
| Diethylether | 99,3 | 23:1 | 1,2:1 |
| Di-iso-propylether | 98,5 | 5:1 | 0,8:1 |
| Di-n-butylether | 97,3 | 9:1 | 1,0:1 |
| Methyl-tert.-butylether | 99,4 | 18:1 | 1,3:1 |
| Anisol | 100 | 24:1 | 1,7:1 |

Die Versuche zeigen, daß das Produkt durch den erfindungsgemäßen Reinigungsschritt in höchster Reinheit erhalten wird. Der Anteil des optisch aktiven Lactids im Feststoff ist gegenüber dem in der Mutterlauge erhöht.

### Beispiel 2

1000 g S-Milchsäure wurden als 50 gew.-%ige wäßrige Lösung bei 10 mbar in 2 h unter Abdestillieren des Wassers auf 150°C erhitzt. Nach Zugabe von 2,5 g Antimontrifluorid wurden bei 10 mbar und 190°C 350 g braungelbes Rohlactid abdestilliert.

Das Rohlactid wurde in 200 g Diethylether gerührt, filtriert und zweimal mit je 50 g Diethylether gewaschen. Nach Trocknung wurden 220 g farblose Nadeln von [L,L]-Lactid mit einer Reinheit von über 99% erhalten, der Rest bestand aus [DD]-und [DL]-Lactid sowie zu weniger als 0,1% aus sonstigen Verunreinigungen (gaschromatographische Analyse).

## Patentansprüche

1. Verfahren zur Herstellung von Glycolid (Ia) oder von Lactiden (Ib) 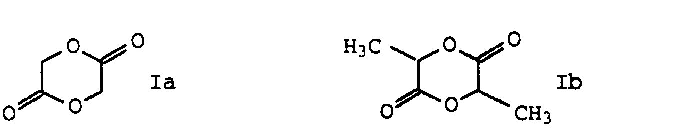 durch thermisch-katalytische Spaltung von Kondensationsprodukten der Glycolsäure bzw. einer α-Hydroxypropionsäure unter destillativer Entfernung von Ia bzw. Ib aus dem Reaktionsgemisch, Überführung des Destillates durch Abkühlen in die feste Phase und Reinigung des so erhaltenen Rohproduktes, dadurch gekennzeichnet, daß man das Rohprodukt mit einem Ether mit 4 bis 12 Kohlenstoffatomen wäscht.

## Claims

1. A process for preparing glycolide (Ia) or lactides (Ib) 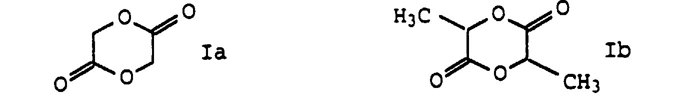 by thermal catalytic cleavage of condensation products of glycolic acid or of an α-hydroxypropionic acid with removal of Ia or Ib from the reaction mixture by distillation, conversion of the distillate to the solid phase by cooling, and purification of the crude product obtained in this way, wherein the crude product is washed with an ether having 4 to 12 carbons.

## Revendications

1. Procédé de préparation de glycolide (Ia) ou de lactides (Ib) 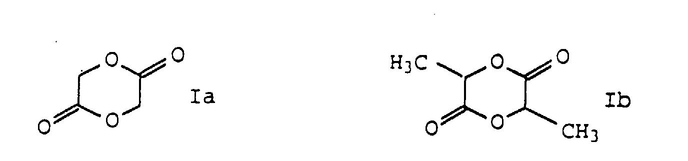 par dissociation thermo-catalytique de produits de condensation de l'acide glycolique ou d'un acide α-hydroxypropionique avec élimination par distillation de Ia ou Ib a partir du mélange réactionnel, transformation du distillat en la phase solide par refroidissement et purification du produit brut ainsi obtenu, caractérisé en ce qu'on lave le produit brut avec un éther à 4-12 atomes de carbone.
